# EUROPEAN PATENT SPECIFICATION

(11) **EP 3 818 942 B1**
(45) Date of publication and mention of the grant of the patent: **06.09.2023**
(21) Application number: 19889027.9
(22) Date of filing: 13.09.2019
(51) Int. Cl.: A61B 8/08, A61B 8/02, A61B 8/00

(54) **ULTRASONIC INSPECTION DEVICE**
ULTRASCHALLPRÜFVORRICHTUNG
DISPOSITIF D'INSPECTION À ULTRASONS

(30) Priority: 28.11.2018 JP 2018222107
(43) Date of publication of application: 12.05.2021
(73) Proprietor: Melody International Ltd., Takamatsu-shi, Kagawa, 761-0301 (JP)
(72) Inventor: OGATA Yhuko, Takamatsu-shi, Kagawa 761-0301 (JP)
(74) Representative: Plougmann Vingtoft a/s
(86) International application number: PCT/JP2019/036218
(87) International publication number: WO 2020/110428

(56) References cited:
- WO-A1-2018/187915
- JP-A- 2008 036 095
- JP-A- 2008 036 095
- JP-A- 2013 027 468
- JP-A- 2016 168 107
- JP-A- 2018 519 964
- US-A1- 2018 049 716
- US-A1- 2018 153 506

## Description

### TECHNICAL FIELD

The present invention relates to ultrasonic inspection apparatuses for inspecting human bodies using ultrasonic waves.

### BACKGROUND OF THE INVENTION

There are conventionally known apparatuses that are capable of identifying states in bodies by transmitting ultrasonic waves. Patent Document 1 discloses an ultrasonic inspection apparatus capable of identifying a fetal heart rate based on ultrasonic Doppler echo signals.

US 2018/153506 A1 discloses A stand-alone continuous cardiac Doppler pulse monitoring patch provides visual and auditory signals that a pulse is detected or not detected in a human subject. The invention is a small patch with a peel-away adhesive surface that is applied to the skin of the subject, preferably near a large artery. The adhesive surface of the patch includes a conductive medium to enhance transmission and reception of ultrasonic waves. The patch includes an integral power source, transmitters and receivers to send and detect reflected ultrasonic waves, a transducer to convert the reflected waves into an electrical signal, a processor to analyze the signal, a light to indicate the presence and strength of a pulse, and a speaker also to indicate the presence and strength of a pulse. The Doppler effect of waves reflecting from blood pumped from a heart is used to detect a pulse in the subject. The presence of a pulse is analyzed by the processor to determine the frequency and strength of blood flow. The processor causes the light to blink at a rate to indicate the frequency of rhythmic blood flow. In a further embodiment, the processor analyzes the strength of the blood flow and causes the light to increase or decrease in intensity to reflect the strength or weakness of the flow. The processor may also drive a speaker to emit sounds, such as beeps, that indicate the frequency and strength of blood flow. The absence of blood flow may be indicated by the absence of light or sound, or by separate light or auditory signals.

JP 2008 036095 A discloses an ultrasonic blood flowmeter determines the frequency of a transmission signal of a connected probe 4. A detection circuit 8 detects Doppler shift frequency component signal out of reflected ultrasonic waves received by the connected probe 4. When the frequency of the transmission signal of the probe 4 is a first frequency, the Doppler shift frequency component signal detected by the detection circuit is acoustically output. On the other hand, when the frequency of the transmission signal of the probe 4 is a second frequency which is lower than the first frequency, the frequency of the detected Doppler shift frequency component signal is multiplied by a frequency multiplication circuit and the frequency-multiplied Doppler shift frequency component signal is acoustically output.

WO 2018/187951 A1 discloses a doppler fetus heart meter, comprising: a housing 100, a main board 200, a loudspeaker 400 and an ultrasonic transducer 500; The loudspeaker 400 and the ultrasonic transducer 500 are installed inside the housing 100; The ultrasonic transducer 500 comprises at least one transduction wafers for generating pulse waves; The loudspeaker 400 and the ultrasonic transducer 500 are in electrical connection to the main board 200, the ultrasonic transducer 500 is disposed at the head end of the housing 100, and the loudspeaker 400 and the main board 200 are disposed at the rear end inside the housing 100. The Doppler fetus heart meter has the characteristics of compact structure and small size by arranging the loudspeaker, the main board and the ultrasonic transducer inside the housing. The Doppler fetus heart meter can reduce the positive feedback from a voice system and reduce the probability of self-excited howling.

US 2018/049716 A1 discloses an enhanced stethoscope device and method for operating the enhanced stethoscope are provided. The enhanced stethoscope device generally operates by providing stethoscope sensors, ultrasonic sensors, and other sensors to obtain a series of measurements about a subject. The series of measurements may be correlated, such as by machine learning, to extract clinically relevant information. Also described are systems and methods for ultrasonic beamsteering by interference of an audio signal with an ultrasonic signal.

### PRIOR ART

### PATENT DOCUMENT

Japanese Translation of PCT International Application Publication No. 2018-527101

### SUMMARY OF DISCLOSURE

### PROBLEMS TO BE SOLVED BY THE DISCLOSURE

Conventional ultrasonic inspection apparatuses generate data for enabling visualization of state in a human body by processing ultrasonic Doppler echo signals generated on the basis of (i) ultrasonic waves emitted from an ultrasonic transducer disposed on a surface of the human body, and (ii) ultrasonic waves reflected within the body. The ultrasonic inspection apparatus may display the generated data on a monitor connected via a network.

The intensity of the ultrasonic waves reflected within the body varies depending on the position of the ultrasonic transducer. Therefore, for checking the state of a specific region, an inspector needs to move the ultrasonic transducer to a position suitable for the inspection of the region to be checked. The conventional ultrasonic inspection apparatuses, if used, suffer from the problem of poor operability in that the inspector needs to move an ultrasonic device while alternating between looking at the image displayed on the monitor connected via the network and looking at the human body to be inspected in order to find an optimum position.

Accordingly, the present invention has been made in view of these points, and an object of the present invention is to improve operability of an ultrasonic inspection apparatus.

### MEANS FOR SOLVING THE PROBLEMS

The invention is defined in claim 1.

The speaker is, for example, provided on a side of a housing of the ultrasonic inspection apparatus opposite to a side on which the ultrasonic sensor unit is provided.

The ultrasonic inspection apparatus may further include a printed circuit board on which the Doppler signal generation unit and the sound signal generation unit are provided, and the speaker may be provided on a side opposite to a side where the ultrasonic sensor unit is provided on the printed circuit board.

The sound signal generation unit may generate the sound signal by multiplying the Doppler signal. The sound signal generation unit may multiply the Doppler signal such that the sound signal is included in a frequency range that includes a resonance frequency of the speaker. The sound signal generation unit may generate the sound signal by multiplying the Doppler signal by a factor of two or more. The sound signal generation unit may generate the sound signal having a magnitude corresponding to an intensity of the reflected ultrasonic wave.

The ultrasonic sensor unit may include a plurality of the ultrasonic reception units, and the sound signal generation unit may generate the sound signal corresponding to a sum of intensities of a plurality of the reflected ultrasonic waves received by the plurality of ultrasonic reception units.

The sound signal generation unit may limit the maximum value of the sound signal to a magnitude where howling does not occur if the intensity of the reflected ultrasonic wave is at a maximum.

The sound signal generation unit may generate the sound signal corresponding to the Doppler signal generated by the Doppler signal generation unit on the basis of a first reflected ultrasonic wave reflected from a fetus in the human body among the first reflected ultrasonic wave and a second reflected ultrasonic wave reflected from a mother's body corresponding to the human body.

The ultrasonic inspection apparatus may further include a display unit provided on a side of the housing of the ultrasonic inspection apparatus opposite to a side on which the ultrasonic sensor unit is provided, the display unit displaying information indicating the intensity of the reflected ultrasonic wave received by the ultrasonic reception unit.

### EFFECT OF THE DISCLOSURE

According to the present invention, improvement in operability of an ultrasonic inspection apparatus can be achieved.

### BRIEF DESCRIPTION OF THE DRAWINGS

FIG. 1 is a diagram showing an external appearance of an ultrasonic inspection apparatus 1.
FIG. 2 is a diagram showing a position where an ultrasonic sensor is disposed inside the ultrasonic inspection apparatus 1.
FIG. 3 is a schematic diagram showing an inner structure of the ultrasonic inspection apparatus 1.
FIG. 4 is a block diagram showing a functional configuration of the ultrasonic inspection apparatus 1.
FIG. 5 is a diagram showing a configuration example of a sound signal generation unit 252.
FIG. 6 is a diagram for explaining a multiplication process in the sound signal generation unit 252.
FIG. 7 is a diagram showing an external appearance of an ultrasonic inspection apparatus 2.
FIG. 8 is a block diagram showing a functional configuration of the ultrasonic inspection apparatus 2.
FIG. 9 is a diagram showing an external appearance of an ultrasonic inspection apparatus 2a.
FIG. 10 is a block diagram showing a functional configuration of an ultrasonic inspection apparatus 3.

### DESCRIPTION OF THE EMBODIMENTS

### <First Embodiment, which is not part of the invention>

### [Outline of Ultrasonic Inspection Apparatus 1]

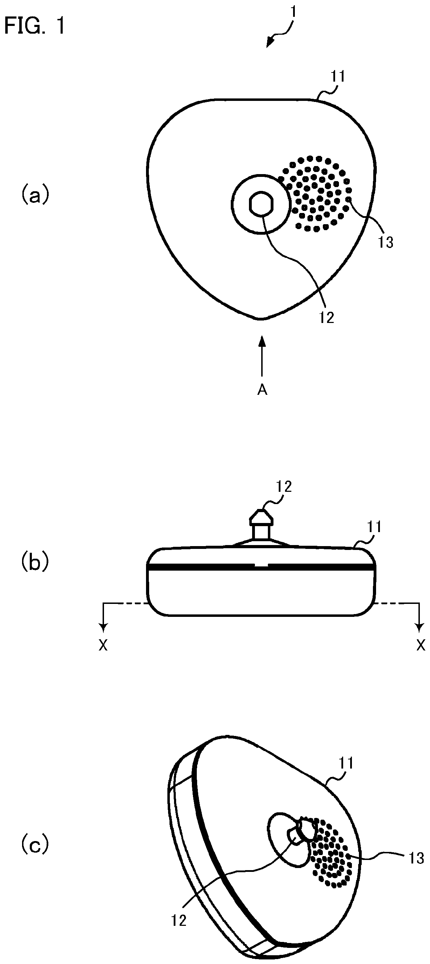

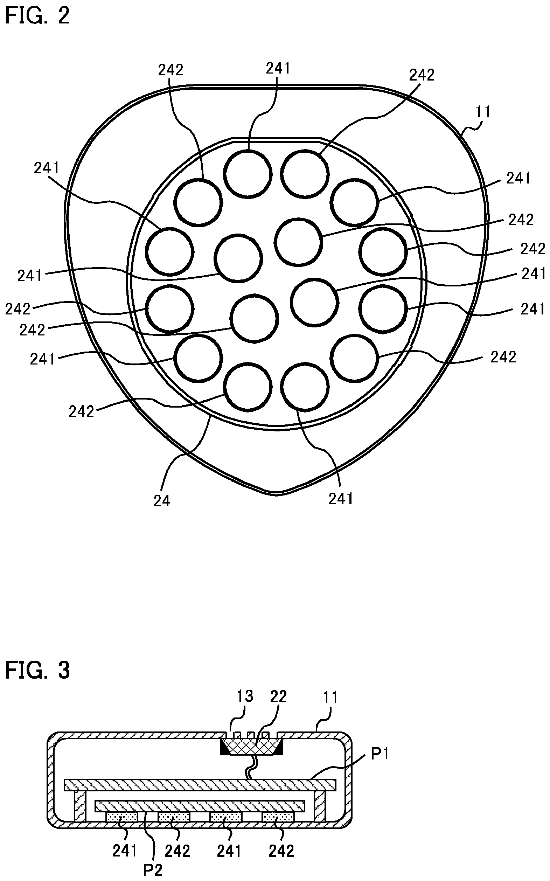

FIG. 1 is a diagram showing an external appearance of an ultrasonic inspection apparatus 1. FIG. 2 is a diagram showing a position where an ultrasonic sensor is disposed inside the ultrasonic inspection apparatus 1, and corresponds to a cross-sectional view taken along line X-X in FIG. 1(b). FIG. 3 is a schematic diagram showing an inner structure of the ultrasonic inspection apparatus 1. FIG. 1(a) is a top view of the ultrasonic inspection apparatus 1 and FIG. 1(b) is a view of the ultrasonic inspection apparatus 1 when seen from the side with the arrow A in FIG. 1(a). FIG. 1(c) is a perspective view of the ultrasonic inspection apparatus 1.

Hereinafter, the configuration of the ultrasonic inspection apparatus 1 will be outlined with reference to FIGS. 1 to 3. The ultrasonic inspection apparatus 1 includes a protrusion 12 provided on a housing 11 and an aperture 13 formed in the housing 11. The protrusion 12 is used, for example, for connecting a belt for fixing the ultrasonic inspection apparatus 1 at an optimum position by a person (hereinafter referred to as a "user") who performs an inspection using the ultrasonic inspection apparatus 1.

The ultrasonic inspection apparatus 1 is an apparatus having an ultrasonic transducer for measuring, for example, a fetal heart rate. When performing an inspection, the user moves a surface of the ultrasonic inspection apparatus 1 on the side opposite to the side on which the protrusion 12 is provided while causing the surface thereof to contact the surface of the human body. The ultrasonic inspection apparatus 1 generates a signal corresponding to the cycle and intensity of the fetal heart rate on the basis of a Doppler signal that is based on a difference between the frequency of an ultrasonic wave transmitted from an ultrasonic transmission device provided inside the housing 11 (hereinafter referred to as a "transmitted ultrasonic wave") and the frequency of a reflected ultrasonic wave, which is an ultrasonic wave resulting from the transmitted ultrasonic wave reflected within the human body.

The ultrasonic inspection apparatus 1 transmits the signal generated based on the Doppler signal to an external terminal via, for example, a wireless communication line. The external terminal may be, for example, a smartphone, a tablet, a computer, or a display. The user can visually confirm, for example, a waveform indicating the cycle and intensity of the heart rate as the signal based on the Doppler signal at the external terminal.

The ultrasonic inspection apparatus 1 also includes a speaker 22 therein, and the speaker emits a sound signal corresponding to the Doppler signal. The sound signal is emitted to the outside of the ultrasonic inspection apparatus 1 via the aperture 13 formed in the housing 11. When inspecting the fetal heart rate, the user can intuitively grasp the fetal state because the user can hear the fetal heart sound generated from the speaker.

The ultrasonic inspection apparatus 1 emits, for example, a sound signal having a magnitude corresponding to the intensity of the reflected ultrasonic wave or the intensity of the Doppler signal. The intensity of the reflected ultrasonic wave is represented, for example, by the maximum voltage of the reflected ultrasonic wave, and the intensity of the Doppler signal is represented, for example, by the maximum voltage of the Doppler signal. With this configuration of the ultrasonic inspection apparatus 1, the closer the ultrasonic inspection apparatus 1 is to the position of the fetal heart, the more loudly the user can hear the fetal heart sound. Therefore, the user can search for a position where the heart sound becomes larger while listening to the heart sound generated by the ultrasonic inspection apparatus 1, and it is therefore easy to move the ultrasonic inspection apparatus 1 to an appropriate position.

As shown in FIG. 2, the ultrasonic inspection apparatus 1 includes a plurality of ultrasonic transmission units 241, which are devices for transmitting ultrasonic waves, and a plurality of ultrasonic reception units 242, which are devices for receiving reflected ultrasonic waves. Provided in the example shown in FIG. 2 are six ultrasonic transmission units 241 and six ultrasonic reception units 242 arranged circumferentially, as well as two ultrasonic transmission units 241 and two ultrasonic reception units 242 arranged on the inward side of the ultrasonic transmission units 241 and the ultrasonic reception units 242 that are arranged circumferentially; however, the number of the ultrasonic transmission units 241 and the ultrasonic reception units 242 of the ultrasonic inspection apparatus 1 is arbitrary. Further, in the example shown in FIG. 2, the ultrasonic transmission units 241 and the ultrasonic reception units 242 are arranged in an alternating manner; however, a plurality of ultrasonic transmission units 241 and a plurality of ultrasonic reception units 242 may be disposed in other modes.

FIG. 3 schematically shows a perspective view of the inside of the ultrasonic inspection apparatus 1 seen from the side with the arrow A in FIG. 1(a). The ultrasonic inspection apparatus 1 includes, inside the housing 11, a printed circuit board P1 on which various electronic components are mounted, and a printed circuit board P2 on which the ultrasonic transmission units 241 and the ultrasonic reception units 242 are mounted. The printed circuit board P1 and the printed circuit board P2 are electrically connected to each other by a connector or cable (not shown).

The speaker 22 is provided on the side of the housing 11 of the ultrasonic inspection apparatus 1, which is opposite to the side on which the ultrasonic transmission units 241 and the ultrasonic reception units 242 are provided. The speaker 22 is electrically connected to the printed circuit board P1 by a connector or cable.

### [Functional Configuration of Ultrasonic Inspection Apparatus 1]

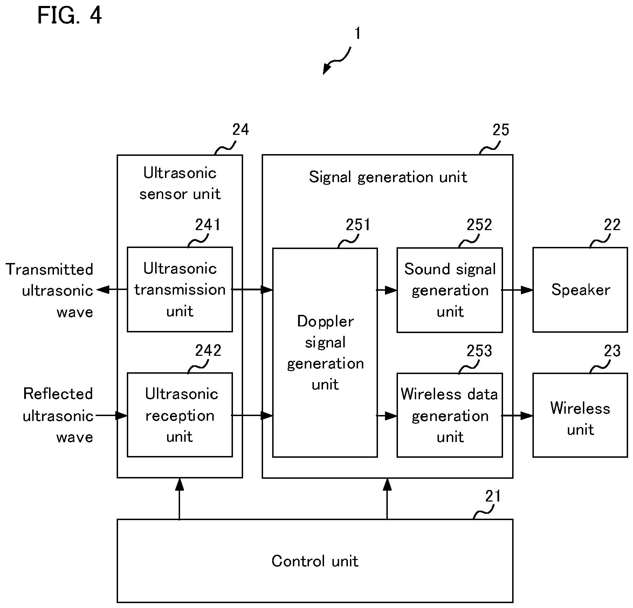

FIG. 4 is a block diagram showing a functional configuration of the ultrasonic inspection apparatus 1. As shown in FIG. 4, the ultrasonic inspection apparatus 1 includes a control unit 21, the speaker 22, a wireless unit 23, an ultrasonic sensor unit 24 and a signal generation unit 25. For example, the control unit 21, the wireless unit 23 and the signal generation unit 25 are mounted on the printed circuit board P1, and the ultrasonic sensor unit 24 is mounted on the printed circuit board P2. The signal generation unit 25 includes a Doppler signal generation unit 251, a sound signal generation unit 252 and a wireless data generation unit 253.

The control unit 21 includes, for example, a central processing unit (CPU), a read-only memory (ROM) and a random access memory (RAM), and the CPU controls the operation of the ultrasonic inspection apparatus 1 by executing a program stored in the ROM. The ultrasonic inspection apparatus 1 may execute at least part of the functions of the signal generation unit 25.

The speaker 22 outputs a sound signal corresponding to the Doppler signal. The speaker 22 is provided on the side opposite to the side where the ultrasonic sensor unit 24 is provided on the printed circuit board P1. By providing the speaker 22 on the side opposite to the side on which the ultrasonic sensor unit 24 is provided, howling is less likely to occur. Further, by providing the speaker 22 on the side opposite to the side on which the ultrasonic sensor unit 24 is provided, the heart rate can be sensed through, in addition to the ears, the vibration due to sound being transmitted to the user's hand.

The speaker 22 can output sound in a predetermined frequency range (i.e. resonance frequencies) more loudly than the sound at other frequencies. The lower limit of the resonant frequency range is the frequency at which the electrical impedance becomes the largest in the audible band. Although the resonance frequency range of the speaker 22 is arbitrary, the resonance frequency range of the speaker 22 of the present embodiment is, for example, from 200 Hz to 10 kHz, and the sound pressure level of the sound output by the speaker 22 can be -10 dB or more in the range from 200 Hz to 10 kHz.

Further, the speaker 22 is provided near the position of the area where a plurality of apertures 13 shown in FIG. 1(a) are provided. Since the speaker 22 is provided at a position between the center position of the ultrasonic inspection apparatus 1 and the outer periphery of the ultrasonic inspection apparatus 1, the user can easily hold the ultrasonic inspection apparatus 1 so that the speaker 22 is not blocked by the palm. For example, the user can easily hear the sound output from the speaker 22 by holding the ultrasonic inspection apparatus 1 such that the little finger is located on the side with the arrow A in FIG. 1(a), thereby exposing at least some of the apertures 13 between the fingers.

The wireless unit 23 transmits data corresponding to the Doppler signal to the external terminal. The wireless unit 23 transmits data indicating, for example, a heart rate waveform. The wireless unit 23 transmits electric waves complying with, for example, Wi-Fi (registered trademark) or Bluetooth (registered trademark) standards.

The ultrasonic sensor unit 24 includes the ultrasonic transmission unit 241 for transmitting ultrasonic waves, and the ultrasonic reception unit 242 for receiving reflected ultrasonic waves, which are ultrasonic waves resulting from the transmitted ultrasonic waves transmitted from the ultrasonic transmission unit 241 being reflected within the human body. The ultrasonic transmission unit 241 transmits an ultrasonic wave of 1 MHz as a frequency suitable for, for example, monitoring the fetal heart rate. The ultrasonic transmission unit 241 starts or stops transmission of ultrasonic waves based on an instruction provided from the control unit 21 in accordance with an operation of the user (e.g., an operation of turning on a power source). The ultrasonic transmission unit 241 inputs a signal based on the transmitted ultrasonic waves to the Doppler signal generation unit 251. The ultrasonic reception unit 242 inputs a signal based on the received reflected ultrasonic waves to the Doppler signal generation unit 251.

The signal generation unit 25 generates various signals. Specifically, the Doppler signal generation unit 251 generates a Doppler signal based on the difference between the frequency of the transmitted ultrasonic wave and the frequency of the reflected ultrasonic wave. The Doppler signal generation unit 251 generates the Doppler signal on the basis of, for example, the autocorrelation function of the reflected ultrasonic wave by using a known autocorrelation method. The Doppler signal is, for example, a signal indicating a value corresponding to a flow velocity of the blood that reflects the transmitted ultrasonic wave. If the frequency of the ultrasonic wave transmitted by the ultrasonic transmission unit 241 is 1 MHz, the frequency of the Doppler signal is, for example, about 100 Hz. The Doppler signal generation unit 251 inputs the generated Doppler signal to the sound signal generation unit 252.

The sound signal generation unit 252 generates a sound signal corresponding to the Doppler signal. The sound signal generation unit 252 generates a sound signal having a magnitude corresponding to, for example, the intensity of the reflected ultrasonic wave or the intensity of the Doppler signal. The sound signal generation unit 252 inputs the generated sound signal to the speaker 22 and the sound based on the sound signal is output from the speaker 22. Since the sound signal generation unit 252 generates the sound signal having a magnitude corresponding to the intensity of the reflected ultrasonic waves or the intensity of the Doppler signal, the user can hear a loud heart rate sound if, for example, the ultrasonic wave is transmitted to a position close to the fetal heart. Therefore, the user can easily adjust the position of the ultrasonic inspection apparatus 1 based on the magnitude of the sound.

The sound signal generation unit 252 may generate a sound signal corresponding to the sum of the intensities of a plurality of reflected ultrasonic waves received by the plurality of ultrasonic reception units 242 or the sum of the intensities of a plurality of Doppler signals based on each of the plurality of reflected ultrasonic waves, so that the magnitude of the sound signal changes with high precision according to the position of the object to be inspected. The sum of the intensities of the plurality of reflected ultrasonic waves or the sum of the intensities of the plurality of Doppler signals is greater when the intensities of the reflected ultrasonic waves detected by each of the plurality of ultrasonic reception units 242 are at an equivalent level than when there is a large difference between the intensities of the reflected ultrasonic waves detected by some ultrasonic reception units 242 among the plurality of the ultrasonic reception units 242 and the intensities of the reflected ultrasonic waves detected by other ultrasonic reception units 242.

Therefore, as the position of the object to be inspected becomes closer to the center position of the ultrasonic inspection apparatus 1 and the variation between the intensities of the reflected ultrasonic waves detected by the plurality of ultrasonic reception units 242 becomes smaller, the sum of the intensities of the plurality of reflected ultrasonic waves or the intensities of the plurality of Doppler signals becomes greater, and the sound speaker 22 therefore emits a louder sound. As a consequence, the user can easily adjust the center position of the ultrasonic inspection apparatus 1 to a desired position (for example, a position close to the fetal heart), while listening to the sound emitted by the speaker 22.

Incidentally, in human auditory characteristics, the sensitivity to sound of frequencies equal to or less than 200 Hz is relatively low. Therefore, if the frequency of the Doppler signal is about 100 Hz and if the Doppler signal of 100 Hz is output as-is from the speaker 22, it is difficult for the user to hear the Doppler signal. Therefore, the sound signal generation unit 252 generates the sound signal by, for example, multiplying the Doppler signal. Specifically, the sound signal generation unit 252 multiplies the Doppler signal so that the sound signal is included in the frequency range including the resonance frequency of the speaker.

More specifically, the sound signal generation unit 252 generates the sound signal by multiplying the Doppler signal by a factor of two or more. If the frequency of the Doppler signal is about 100 Hz, then the speaker 22 can generate a sound signal having a frequency of about 200 Hz or more by having the sound signal generation unit 252 multiply the Doppler signal by a factor of two or more. Therefore, the user can easily hear the heart rate sound that is based on the Doppler signal. In particular, if the resonance frequency range of the speaker 22 is between 200 Hz and 10 kHz, it is preferred that the sound signal generation unit 252 multiplies the Doppler signal by a factor of two or three.

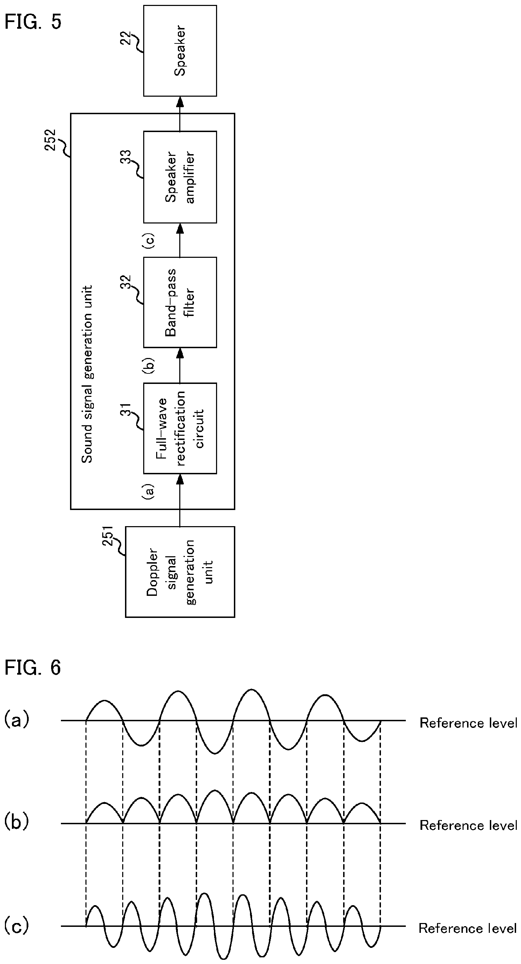

FIG. 5 is a diagram showing a configuration example of the sound signal generation unit 252. FIG. 6 is a diagram for explaining the multiplication process in the sound signal generation unit 252. As shown in FIG. 5, the sound signal generation unit 252 includes a full-wave rectification circuit 31, a band-pass filter 32 and a speaker amplifier 33.

FIG. 6(a) shows an exemplary waveform of the Doppler signal input from the Doppler signal generation unit 251 to the full-wave rectification circuit 31. FIG. 6(b) shows an exemplary waveform of the signal output from the full-wave rectification circuit 31. FIG. 6(c) shows an exemplary waveform of the signal output from the band-pass filter 32. It should be noted that the waveforms shown in FIG. 6 are schematic waveforms and are different from the actual waveforms.

By subjecting the Doppler signal input from the Doppler signal generation unit 251 to full-wave rectification, the full-wave rectification circuit 31 converts the signal below the reference level to a signal equal to or above the reference level. The full-wave rectification circuit 31 inputs the converted signal to the band-pass filter 32.

The bandpass filter 32 is a filter that passes, among the frequency components included in the signal input from the full-wave rectification circuit 31, a predetermined frequency component included in the audible range of the user; and does not pass other frequency components. The band-pass filter 32 passes the frequency component between, for example, 200 Hz and 10 kHz, to the speaker amplifier 33. By extracting the frequency component between 200 Hz and 10 kHz from the signal shown in FIG. 6(b), a signal obtained by multiplying the Doppler signal shown in FIG. 6(a) by a factor of two is generated as shown in FIG. 6(c).

The occurrence of howling due to the vibration of the ultrasonic sensor unit 24 caused by the vibration emitted by the speaker 22 is also prevented by the speaker 22 outputting the sound having the frequency obtained by multiplying the frequency of the Doppler signal. It should be noted that, in order to prevent the occurrence of howling, the sound signal generation unit 252 may limit the maximum value of the sound signal to a magnitude where howling does not occur if the intensity of the reflected ultrasonic wave is at a maximum. For example, the speaker 22 generates a sound signal having a magnitude corresponding to the intensity of the reflected ultrasonic wave if the intensity of the reflected ultrasonic wave is less than a threshold value, and it generates a sound signal corresponding to the intensity of the reflected ultrasonic wave equal to the threshold if the intensity of the reflected ultrasonic wave is equal to or greater than the threshold value.

### [Separation of Fetal and Maternal Heart Sounds]

When the user inspects the fetal heart rate using the ultrasonic inspection apparatus 1, the reflected ultrasonic waves received by the ultrasonic reception units 242 include a signal synchronized with the fetal heart rate and a signal synchronized with the heart rate of the mother. The sound signal generation unit 252 selectively generates a sound signal corresponding to the fetal heart rate in order to make it easier for the user to hear the fetal heart rate sound. Specifically, the reflected ultrasonic waves includes a first reflected ultrasonic wave reflected from the fetus in the human body and a second reflected ultrasonic wave reflected from the mother's body, and the sound signal generation unit 252 generates a sound signal corresponding to the Doppler signal generated by the Doppler signal generation unit 251 based on the first reflected ultrasonic wave.

Among the sound signals generated based on the Doppler signals, the sound signal generation unit 252 generates a sound signal to be output to the speaker 22 by, for example, removing a frequency component corresponding to a cycle within a predetermined range with respect to the mother's average heart rate cycle (for example, a cycle within a range not including the fetus's average heart rate cycle) and by extracting a frequency component corresponding to a cycle within a predetermined range with respect to the fetus's average heart rate cycle. The sound signal generation unit 252 may generate a sound signal to be output to the speaker 22 by making the amplification factor for the frequency component corresponding to the fetus's average heart rate cycle larger than the amplification factor for the frequency component corresponding to the mother's average heart rate cycle.

### [Wireless Transmission of Heart Rate Signal]

The wireless data generation unit 253 generates wireless data for the wireless unit 23 to transmit to an external terminal on the basis of the Doppler signal. The wireless data generation unit 253 converts, for example, the Doppler signal into a digital signal and generates the wireless data having a frame format defined by a predetermined wireless communication protocol. The wireless data generation unit 253 inputs the generated wireless data to the wireless unit 23.

### [Effects of Ultrasonic Inspection Apparatus 1 According to First Embodiment]

As described above, the ultrasonic inspection apparatus 1 emits, with the speaker 22, a sound signal corresponding to the Doppler signal based on the difference between the frequency of the transmitted ultrasonic wave and the frequency of the reflected ultrasonic wave. Due to the ultrasonic inspection apparatus 1 emitting the sound signal in this manner, the user can hear the sound indicating the state of the region to be inspected while performing the inspection using the ultrasonic inspection apparatus 1. Accordingly, for example, when the user seeks to check the fetal state, the user can hear the fetal heart rate sound while watching the mother's body which is in contact with the ultrasonic inspection apparatus 1, and the user can therefore easily search for the optimum position and operability is thereby improved.

In particular, the ultrasonic inspection apparatus 1 can emit sound, the magnitude of which changes according to the intensity of the reflected ultrasonic wave or the intensity of the Doppler signal based on the reflected ultrasonic wave, and thus, a louder sound is emitted when the ultrasonic inspection apparatus 1 is closer to the position of the fetus. Accordingly, the user can search for the optimum position by referring to the loudness of the sound.

Further, the ultrasonic inspection apparatus 1 generates a sound signal by multiplying the Doppler signal so that sound having a frequency matching the resonance frequency of the speaker 22 can be emitted. This configuration of the ultrasonic inspection apparatus 1 enables the user to easily hear the fetal heart rate sound.

### <Second Embodiment>

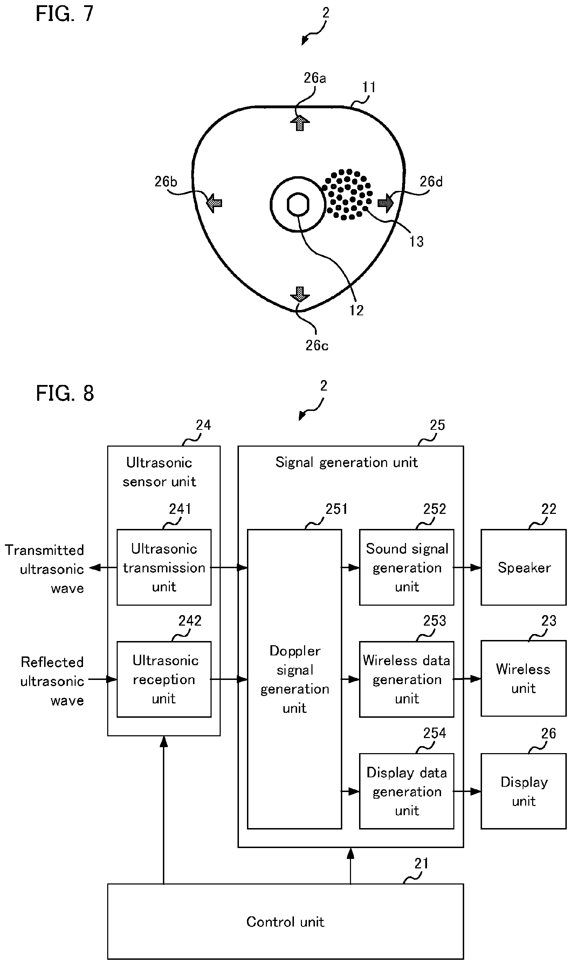

FIG. 7 is a diagram showing an external appearance of an ultrasonic inspection apparatus 2 according to the second embodiment. The ultrasonic inspection apparatus 2 differs from the ultrasonic inspection apparatus 1 shown in FIG. 1 in that it further includes a display unit 26 on the side on which the protrusion 12 is provided, i.e. on the side opposite to the side on which the ultrasonic transmission units 241 and the ultrasonic reception units 242 are provided, but it is equivalent to the ultrasonic inspection apparatus 1 in other respects. The display unit 26 displays information indicating the intensity of the reflected ultrasonic waves received by the ultrasonic reception units 242. The display unit 26 is provided on the side of the housing 11 of the ultrasonic inspection apparatus 1 opposite to the side on which the ultrasonic sensor unit 24 is provided.

The display unit 26 includes a plurality of light-emitting elements provided at least at four positions outward from the center position of the housing 11 of the ultrasonic inspection apparatus 2. In the example shown in FIG. 7, display units 26a, 26b, 26c, 26d, which are light-emitting areas in the form of an arrow, are respectively provided on the back side, the left side, the front side, and the right side with respect to the center position of the housing 11, as the plurality of light-emitting elements. The brightness of each of the display units 26a, 26b, 26c, 26d changes in synchronization with the change in the intensity of the sound signal. For example, the larger the intensity of the sound signal, the brighter each of the display units 26a, 26b, 26c, 26d is.

FIG. 8 is a block diagram showing a functional configuration of the ultrasonic inspection apparatus 2. The block diagram shown in FIG. 8 differs from the block diagram of the ultrasonic inspection apparatus 1 shown in FIG. 4 in that the signal generation unit 25 further includes a display data generation unit 254.

The display data generation unit 254 generates display data based on the reflected ultrasonic waves. The display data generation unit 254 generates, for example, display data showing a size corresponding to the intensity of the reflected ultrasonic waves. The display data generation unit 254 inputs the generated display data to the display unit 26, and the display unit 26 displays information corresponding to the display data. The display data generation unit 254 may also generate display data based on the Doppler signals.

The display data generation unit 254 generates, for example, display data for displaying information corresponding to the intensity of the Doppler signals on the display unit 26. Specifically, the display data generation unit 254 generates display data corresponding to higher brightness as the intensity of the Doppler signals becomes larger. With this configuration of the display data generation unit 254, the closer the ultrasonic inspection apparatus 2 is to, for example, the position of the fetus, the brighter the display shown by the display unit 26. As a consequence, the user can easily determine whether or not the ultrasonic inspection apparatus 2 is close to the position of the fetus.

The display data generation unit 254 may generate display data with which the size changes according to a change in the intensity of the Doppler signals. In this case, as the brightness of the display unit 26 changes in synchronization with, for example, a fetal heart rate, the user can easily grasp the state of the heart rate.

The Doppler signal generation unit 251 may generate a plurality of Doppler signals based on the difference between the frequencies of the transmitted ultrasonic waves and the frequencies of the reflected ultrasonic waves received by each of the plurality of ultrasonic reception units, and the display data generation unit 254 may generate display data corresponding to the sum of the intensities of the plurality of Doppler signals. At this time, the display data generation unit 254 may generate the display data based on the intensities of the plurality of Doppler signals corresponding to the fetal heart rate.

For example, the display data generation unit 254 generates display data corresponding to the sum of the intensities of a plurality of reflected ultrasonic waves received by the plurality of ultrasonic reception units 242, or the sum of the intensities of a plurality of Doppler signals based on the plurality of reflected ultrasonic waves. The sum of the intensities of the plurality of reflected ultrasonic waves or the sum of the intensities of the plurality of Doppler signals is greater when the intensities of the reflected ultrasonic waves detected by each of the plurality of ultrasonic reception units 242 are at an equal level than when there is a large difference between the intensities of the reflected ultrasonic waves detected by some ultrasonic reception units 242 among the plurality of the ultrasonic reception units 242 and the intensities of the reflected ultrasonic waves detected by other ultrasonic reception units 242.

Therefore, as the position of the object to be inspected becomes closer to the center position of the ultrasonic inspection apparatus 2 and the variation between the intensities of the reflected ultrasonic waves detected by the plurality of ultrasonic reception units 242 becomes smaller, the sum of the intensities of the plurality of reflected ultrasonic waves or the intensities of the plurality of Doppler signals becomes greater, and the display unit 26 emits brighter light. As a consequence, the user can easily adjust the center position of the ultrasonic inspection apparatus 2 to a desired position (for example, a position close to the fetal heart), while visually recognizing the light emitted from the display unit 26.

The display data generation unit 254 may generate display data indicating an orientation identified based on the intensities of the reflected ultrasonic waves received by each of the plurality of ultrasonic reception units 242. The display data generation unit 254 generates, for example, display data for making the display unit 26 brighter, which is provided at a position corresponding to the ultrasonic reception unit 242 that received a relatively strong reflected ultrasonic wave, from among the plurality of ultrasonic reception units 242.

The display data generation unit 254 may generate display data indicating an orientation identified on the basis of the intensities of the Doppler signals based on the reflected ultrasonic waves received by each of the plurality of ultrasonic reception units 242. The display data generation unit 254 generates, for example, display data for making the display unit 26 brighter, which is provided at a position corresponding to the ultrasonic reception unit 242, in which relatively strong Doppler signals are generated, from among the plurality of ultrasonic reception units 242. At this time, the display data generation unit 254 may generate the display data based on the intensities of the plurality of Doppler signals corresponding to the fetal heart rate.

For example, the display data generation unit 254 generates display data for recommending moving the ultrasonic inspection apparatus 2 to a position where the ultrasonic reception unit 242 which received a relatively large-intensity reflected ultrasonic wave from among the plurality of ultrasonic reception units 242 is provided. Specifically, if the intensity of the Doppler signal based on the reflected ultrasonic waves received by the ultrasonic reception units 242 disposed on the right side with respect to the center position of the ultrasonic inspection apparatus 2 is relatively large, the display data generation unit 254 makes the display unit 26 provided on the right side (the display unit 26d in the example shown in FIG. 7) of the ultrasonic inspection apparatus 2 brighter than the other display units 26. The display data generation unit 254 may generate display data causing the display unit 26 to blink on the side to which the ultrasonic inspection apparatus 2 is to be moved.

This configuration of the display data generation unit 254 enables the user to grasp the side (e.g., the back side, the left side, the front side, or the right side), with respect to the ultrasonic inspection apparatus 2, on which the position where the fetal heart rate is strongly detected is located. Therefore, the user can easily move the ultrasonic inspection apparatus 2 to a position where the fetal heart rate can be easily detected.

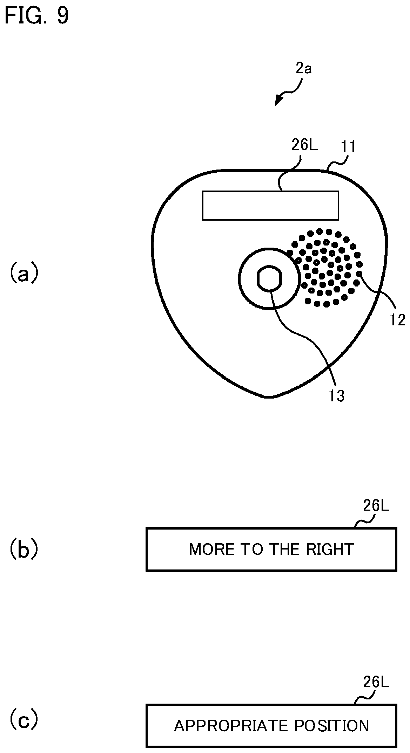

FIG. 9 is a diagram showing an external appearance of an ultrasonic inspection apparatus 2a, which is a variation example of the ultrasonic inspection apparatus 2 according to the second embodiment. The ultrasonic inspection apparatus 2a differs from the ultrasonic inspection apparatus 2 in that a display unit 26L is provided instead of the display unit 26 in the ultrasonic inspection apparatus 2 shown in FIG. 7. The display unit 26L is, for example, a liquid crystal display and can display text or images based on the display data generated by the display data generation unit 254. Specifically, based on the intensities of the reflected ultrasonic waves received by the ultrasonic reception units 242 or on the intensities of the Doppler signals generated by the Doppler signal generation unit 251, the display data generation unit 254 generates display data including information for assisting in moving the ultrasonic inspection apparatus 2a so that the intensities of the reflected ultrasonic waves or the Doppler signals increase.

FIG. 9(b) and FIG. 9(c) show examples of the text displayed on the display unit 26L. FIG. 9(b) illustrates a case where the display unit 26L displays "MORE TO THE RIGHT" as a text displayed if the intensity of the reflected ultrasonic waves received by the ultrasonic reception units 242 disposed on the right side of the ultrasonic inspection apparatus 2a or the Doppler signals based on such reflected ultrasonic waves is relatively large. A user who visually recognizes this text can move the ultrasonic inspection apparatus 2a to the right to bring the center position of the ultrasonic inspection apparatus 2a closer to the position of the object to be inspected.

FIG. 9(c) illustrates a case where "APPROPRIATE POSITION" is displayed on the display unit 26L as a text displayed if the position of the ultrasonic inspection apparatus 2a is appropriate. If, for example, the intensities of the plurality of reflected ultrasonic waves received by each of the plurality of ultrasonic reception units 242 are equal to or greater than a predetermined value and if the variation in the intensities of the plurality of reflected ultrasonic waves is within a predetermined range, the display data generation unit 254 generates display data indicating that the position of the ultrasonic inspection apparatus 2a is appropriate. The display data generation unit 254 may also generate display data indicating that the position of the ultrasonic inspection apparatus 2a is appropriate if the intensities of the plurality of Doppler signals based on the reflected ultrasonic waves received by each of the plurality of ultrasonic reception units 242 are equal to or greater than a predetermined value and if the variation in the intensities of the plurality of Doppler signals is within a predetermined range. This configuration of the display data generation unit 254 enables the user to easily maintain the position of the ultrasonic inspection apparatus 2a if the position of the ultrasonic inspection apparatus 2a is appropriate.

### [Effects of Ultrasonic Inspection Apparatus 2 According to Second Embodiment]

As described above, the ultrasonic inspection apparatus 2 displays information corresponding to the display data generated based on the reflected ultrasonic waves. The ultrasonic inspection apparatus 2 causes, for example, light-emitting elements to emit light at a brightness corresponding to the intensity of the reflected ultrasonic waves or to the intensity of the Doppler signals based on the reflected ultrasonic waves, or causes a display to display information indicating content corresponding to the intensity. This configuration of the ultrasonic inspection apparatus 2 enables the user to grasp whether or not the position of the ultrasonic inspection apparatus 2 is appropriate while operating the ultrasonic inspection apparatus 2, and operability is thereby improved. In particular, the ultrasonic inspection apparatus 2 can display the information indicating the orientation of the appropriate position, and therefore, if the user notices that the fetus has not been successfully measured due to the fetus moving inside the womb after starting the measurement with the ultrasonic inspection apparatus 2 at a fixed position, the ultrasonic inspection apparatus 2 can be moved to the appropriate orientation.

### [Third Embodiment]

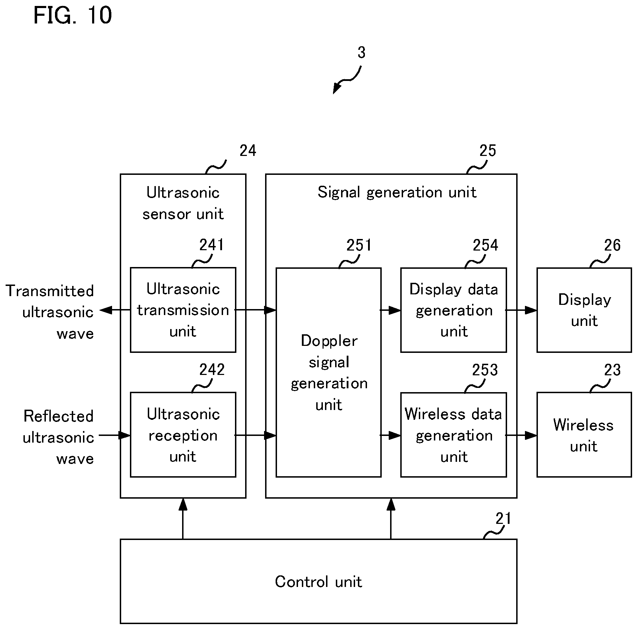

FIG. 10 is a block diagram showing a functional configuration of an ultrasonic inspection apparatus 3 according to the third embodiment. The ultrasonic inspection apparatus 3 differs from the ultrasonic inspection apparatus 2 shown in FIG. 8 in that the ultrasonic inspection apparatus 3 does not include a sound signal generation unit 252, but it is equivalent to the ultrasonic inspection 2 in other respects. The ultrasonic inspection apparatus 3 outputs the display data based on the reflected ultrasonic waves or the Doppler signals, without outputting the sound signal. The contents of the display data generated by the display data generation unit 254 are similar to those of the ultrasonic inspection apparatus 2 according to the second embodiment. Even if the ultrasonic inspection apparatus 3 does not output a sound signal, the user can still recognize how to move the ultrasonic inspection apparatus 3 based on the displayed information, and operability is thereby improved.

The present invention is described using the embodiments of the present invention. The technical scope of the present invention is, however, not limited to the scope described in the above embodiments and various variations and modifications are possible within the scope of the gist thereof. For example, the specific embodiments of the distribution and integration of the apparatuses are not limited to the above embodiments, and all or part thereof can be configured with any unit in a functionally or physically dispersed or integrated manner. Further, new embodiments generated by arbitrary combinations of a plurality of embodiments are also included in the embodiments of the present invention. Further, the effects of the new embodiments brought by the combinations also have the effects of the original embodiments.

### REFERENCES

- 1, 2, 3: ultrasonic inspection apparatus
- 11: housing
- 12: protrusion
- 13: aperture
- 21: control unit
- 22: speaker
- 23: wireless unit
- 24: ultrasonic sensor unit
- 25: signal generation unit
- 26: display unit
- 31: full-wave rectification circuit
- 32: band-pass filter
- 33: speaker amplifier
- 241: ultrasonic transmission unit
- 242: ultrasonic reception unit
- 251: Doppler signal generation unit
- 252: sound signal generation unit
- 253: wireless data generation unit
- 254: display data generation unit

## Claims

1. An ultrasonic inspection apparatus (1) comprising:
an ultrasonic sensor unit (24) including an ultrasonic transmission unit (241) for transmitting an ultrasonic wave and an ultrasonic reception unit (242) for receiving a reflected ultrasonic wave, the reflected ultrasonic wave being an ultrasonic wave resulting from the transmitted ultrasonic wave that is transmitted by the ultrasonic transmission unit (241) and reflected in a human body;
a Doppler signal generation unit (251) for generating a Doppler signal based on a difference between a frequency of the transmitted ultrasonic wave and a frequency of the reflected ultrasonic wave;
a sound signal generation unit (252) for generating a sound signal corresponding to the Doppler signal; and
a speaker (22) for outputting the sound signal,
**characterized in that**
the ultrasonic inspection apparatus (1) comprises:
a display unit comprising a plurality of light-emitting elements whose brightness change in synchronization with the change in the intensity of the sound signal and that are provided at least at four positions outward from the center position of the housing (11) of the ultrasonic inspection apparatus (1).

2. The ultrasonic inspection apparatus (1) according to claim 1, wherein
the speaker (22) is provided on a side of a housing of the ultrasonic inspection apparatus opposite to a side on which the ultrasonic sensor unit is provided.

3. The ultrasonic inspection apparatus (1) according to claim 1 or 2, further comprising:
a printed circuit board (P1) on which the Doppler signal generation unit (251) and the sound signal generation unit (252) are provided, wherein
the speaker (22) is provided on a side opposite to a side where the ultrasonic sensor unit (24) is provided on the printed circuit board (P1).

4. The ultrasonic inspection apparatus (1) according to any one of claims 1 to 3, wherein
the sound signal generation unit (252) generates the sound signal by multiplying the Doppler signal.

5. The ultrasonic inspection apparatus (1) according to claim 4, wherein
the sound signal generation unit (252) multiplies the Doppler signal such that the sound signal is included in a frequency range that includes a resonance frequency of the speaker (22).

6. The ultrasonic inspection apparatus (1) according to claim 4 or 5, wherein
the sound signal generation unit (252) generates the sound signal by multiplying the Doppler signal by a factor of two or more.

7. The ultrasonic inspection apparatus (1) according to any one of claims 1 to 6, wherein
the sound signal generation unit (252) generates the sound signal having a magnitude corresponding to an intensity of the reflected ultrasonic wave.

8. The ultrasonic inspection apparatus (1) according to claim 7, wherein
the ultrasonic sensor unit (24) includes a plurality of the ultrasonic reception units (242), and
the sound signal generation unit (252) generates the sound signal corresponding to a sum of intensities of a plurality of the reflected ultrasonic waves received by the plurality of ultrasonic reception units (242).

9. The ultrasonic inspection apparatus (1) according to claim 7 or 8, wherein
the sound signal generation unit (252) limits the maximum value of the sound signal to a magnitude at which howling does not occur if the intensity of the reflected ultrasonic wave is at a maximum.

10. The ultrasonic inspection apparatus (1) according to any one of claims 1 to 9, wherein
the sound signal generation unit (252) generates the sound signal corresponding to the Doppler signal generated by the Doppler signal generation unit (251) on the basis of a first reflected ultrasonic wave reflected from a fetus in the human body among the first reflected ultrasonic wave and a second reflected ultrasonic wave reflected from a mother's body corresponding to the human body.

11. The ultrasonic inspection apparatus (1) according to any one of claims 1 to 10, wherein
the display unit (26) is provided on a side of the housing (11) of the ultrasonic inspection apparatus (1) opposite to a side on which the ultrasonic sensor unit (24) is provided.

12. The ultrasonic inspection apparatus (1) according to any one of claims 1 to 11, further comprising:
a display data generation unit (254) that generates display data for making the display unit (26) brighter, which is provided at a position corresponding to the ultrasonic reception unit (242) that received a relatively strong reflected ultrasonic wave, from among the plurality of ultrasonic reception units (242).

13. The ultrasonic inspection apparatus (1) according to claim 12, wherein
the display data generation unit (254) generates the display data for recommending moving the ultrasonic inspection apparatus (1) to a position where the ultrasonic reception unit (242) which received a relatively large-intensity reflected ultrasonic wave from among the plurality of ultrasonic reception units (242).

## Patentansprüche

1. Ultraschallprüfvorrichtung (1), umfassend:
eine Ultraschallsensoreinheit (24), die eine Ultraschallsendeeinheit (241) zum Senden einer Ultraschallwelle und eine Ultraschallempfangseinheit (242) zum Empfangen einer reflektierten Ultraschallwelle aufweist, wobei die reflektierte Ultraschallwelle eine Ultraschallwelle ist, die sich aus der gesendeten Ultraschallwelle ergibt, die von der Ultraschallsendeeinheit (241) gesendet und in einem menschlichen Körper reflektiert wird;
eine Dopplersignalerzeugungseinheit (251) zum Erzeugen eines Dopplersignals auf der Grundlage einer Differenz zwischen einer Frequenz der gesendeten Ultraschallwelle und einer Frequenz der reflektierten Ultraschallwelle;
eine Schallsignalerzeugungseinheit (252) zum Erzeugen eines Schallsignals, das dem Dopplersignal entspricht; und
einen Lautsprecher (22) zum Ausgeben des Schallsignals,
**dadurch gekennzeichnet, dass**
die Ultraschallprüfvorrichtung (1) umfasst:
eine Anzeigeeinheit, die eine Vielzahl von lichtemittierenden Elementen umfasst, deren Helligkeit sich synchron mit der Änderung der Intensität des Schallsignals ändert und die an mindestens vier Positionen außerhalb der Mittelposition des Gehäuses (11) der Ultraschallprüfvorrichtung (1) bereitgestellt ist.

2. Ultraschallprüfvorrichtung (1) nach Anspruch 1, wobei
der Lautsprecher (22) an einer Seite eines Gehäuses der Ultraschallprüfvorrichtung bereitgestellt ist, die einer Seite gegenüberliegt, an der die Ultraschallsensoreinheit bereitgestellt ist.

3. Ultraschallprüfvorrichtung (1) nach Anspruch 1 oder 2, weiter umfassend:
eine Leiterplatte (P1), auf der die Dopplersignalerzeugungseinheit (251) und die Schallsignalerzeugungseinheit (252) bereitgestellt sind, wobei
der Lautsprecher (22) auf einer Seite bereitgestellt ist, die einer Seite gegenüberliegt, auf der die Ultraschallsensoreinheit (24) auf der Leiterplatte (P1) bereitgestellt ist.

4. Ultraschallprüfvorrichtung (1) nach einem der Ansprüche 1 bis 3, wobei
die Schallsignalerzeugungseinheit (252) das Schallsignal durch Multiplikation des Dopplersignals erzeugt.

5. Ultraschallprüfvorrichtung (1) nach Anspruch 4, wobei
die Schallsignalerzeugungseinheit (252) das Dopplersignal multipliziert, so dass das Schallsignal in einem Frequenzbereich enthalten ist, der eine Resonanzfrequenz des Lautsprechers (22) aufweist.

6. Ultraschallprüfvorrichtung (1) nach Anspruch 4 oder 5, wobei
die Schallsignalerzeugungseinheit (252) das Schallsignal durch Multiplikation des Dopplersignals mit einem Faktor von zwei oder mehr erzeugt.

7. Ultraschallprüfvorrichtung (1) nach einem der Ansprüche 1 bis 6, wobei
die Schallsignalerzeugungseinheit (252) das Schallsignal mit einer Größe erzeugt, die einer Intensität der reflektierten Ultraschallwelle entspricht.

8. Ultraschallprüfvorrichtung (1) nach Anspruch 7, wobei
die Ultraschallsensoreinheit (24) eine Vielzahl der Ultraschallempfangseinheiten (242) aufweist, und
die Schallsignalerzeugungseinheit (252) das Schallsignal erzeugt, das einer Summe von Intensitäten einer Vielzahl von reflektierten Ultraschallwellen entspricht, die von der Vielzahl von Ultraschallempfangseinheiten (242) empfangen wird.

9. Ultraschallprüfvorrichtung (1) nach Anspruch 7 oder 8, wobei
die Schallsignalerzeugungseinheit (252) den Maximalwert des Schallsignals auf eine Größe begrenzt, bei der kein Heulen auftritt, wenn die Intensität der reflektierten Ultraschallwelle maximal ist.

10. Ultraschallprüfvorrichtung (1) nach einem der Ansprüche 1 bis 9, wobei
die Schallsignalerzeugungseinheit (252) das Schallsignal, das dem von der Dopplersignalerzeugungseinheit (251) erzeugten Dopplersignal entspricht, auf der Grundlage einer ersten reflektierten Ultraschallwelle, die von einem Fötus in dem menschlichen Körper reflektiert wird, unter der ersten reflektierten Ultraschallwelle und einer zweiten reflektierten Ultraschallwelle, die von einem dem menschlichen Körper entsprechenden Körper der Mutter reflektiert wird, erzeugt.

11. Ultraschallprüfvorrichtung (1) nach einem der Ansprüche 1 bis 10, wobei
die Anzeigeeinheit (26) auf einer Seite des Gehäuses (11) der Ultraschallprüfvorrichtung (1) bereitgestellt ist, die einer Seite gegenüberliegt, auf der die Ultraschallsensoreinheit (24) bereitgestellt ist.

12. Ultraschallprüfvorrichtung (1) nach einem der Ansprüche 1 bis 11, weiter umfassend:
eine Anzeigedatenerzeugungseinheit (254), die Anzeigedaten erzeugt, um die Anzeigeeinheit (26) heller zu machen, die an einer Position bereitgestellt ist, die der Ultraschallempfangseinheit (242), die eine relativ stark reflektierte Ultraschallwelle empfangen hat, aus der Vielzahl von Ultraschallempfangseinheiten (242) entspricht.

13. Ultraschallprüfvorrichtung (1) nach Anspruch 12, wobei
die Anzeigedatenerzeugungseinheit (254) die Anzeigedaten erzeugt, um zu empfehlen, die Ultraschallprüfvorrichtung (1) an eine Position zu bewegen, an der die Ultraschallempfangseinheit (242), die eine reflektierte Ultraschallwelle relativ großer Intensität empfangen hat, aus der Vielzahl von Ultraschallempfangseinheiten (242).

## Revendications

1. Appareil d'inspection par ultrasons (1) comprenant :
une unité de capteur ultrasonique (24) comportant une unité d'émission ultrasonique (241) pour émettre une onde ultrasonique et une unité de réception ultrasonique (242) pour recevoir une onde ultrasonique réfléchie, l'onde ultrasonique réfléchie étant une onde ultrasonique résultant de l'onde ultrasonique qui est transmise par l'unité d'émission ultrasonique (241) et réfléchie dans un corps humain ;
une unité de génération de signal Doppler (251) pour générer un signal Doppler sur la base d'une différence entre une fréquence de l'onde ultrasonique transmise et une fréquence de l'onde ultrasonique réfléchie ;
une unité de génération de signal sonore (252) pour générer un signal sonore correspondant au signal Doppler ; et
un haut-parleur (22) pour émettre le signal sonore,
**caractérisé en ce que**
l'appareil d'inspection par ultrasons (1) comprend :
une unité d'affichage comprenant une pluralité d'éléments émetteurs de lumière dont la luminosité varie en synchronisation avec le changement d'intensité du signal sonore et qui sont prévus au niveau d'au moins quatre positions à l'extérieur de la position centrale du boîtier (11) de l'appareil d'inspection par ultrasons (1).

2. Appareil d'inspection par ultrasons (1) selon la revendication 1, dans lequel
le haut-parleur (22) est placé sur un côté du boîtier de l'appareil d'inspection par ultrasons opposé au côté où se trouve l'unité de capteur ultrasonique.

3. Appareil d'inspection par ultrasons (1) selon la revendication 1 ou 2, comprenant en outre :
une carte de circuit imprimé (P1) sur laquelle l'unité de génération de signal Doppler (251) et l'unité de génération de signal sonore (252) sont installées, dans lequel
le haut-parleur (22) est placé sur un côté opposé à un côté où l'unité de capteur ultrasonique (24) est placée sur la carte de circuit imprimé (P1).

4. Appareil d'inspection par ultrasons (1) selon l'une des revendications 1 à 3, dans lequel
l'unité de génération de signal sonore (252) génère le signal sonore en multipliant le signal Doppler.

5. Appareil d'inspection par ultrasons (1) selon la revendication 4, dans lequel
l'unité de génération de signal sonore (252) multiplie le signal Doppler de manière à ce que le signal sonore soit inclus dans une gamme de fréquences qui inclut une fréquence de résonance du haut-parleur (22).

6. Appareil d'inspection par ultrasons (1) selon la revendication 4 ou 5, dans lequel
l'unité de génération de signal sonore (252) génère le signal sonore en multipliant le signal Doppler par un facteur de deux ou plus.

7. Appareil d'inspection par ultrasons (1) selon l'une des revendications 1 à 6, dans lequel
l'unité de génération de signal sonore (252) génère le signal sonore dont l'amplitude correspond à une intensité de l'onde ultrasonique réfléchie.

8. Appareil d'inspection par ultrasons (1) selon la revendication 7, dans lequel
l'unité de capteur ultrasonique (24) comporte une pluralité des unités de réception ultrasonique (242), et
l'unité de génération de signal sonore (252) génère le signal sonore correspondant à une somme d'intensités d'une pluralité des ondes ultrasoniques réfléchies reçues par la pluralité d'unités de réception ultrasonique (242).

9. Appareil d'inspection par ultrasons (1) selon la revendication 7 ou 8, dans lequel
l'unité de génération de signal sonore (252) limite la valeur maximale du signal sonore à une amplitude à laquelle il n'y a pas de sifflement si l'intensité de l'onde ultrasonique réfléchie est maximale.

10. Appareil d'inspection par ultrasons (1) selon l'une des revendications 1 à 9, dans lequel
l'unité de génération de signal sonore (252) génère le signal sonore correspondant au signal Doppler généré par l'unité de génération de signal Doppler (251) sur la base d'une première onde ultrasonique réfléchie par un foetus dans le corps humain parmi la première onde ultrasonique réfléchie et d'une deuxième onde ultrasonique réfléchie par le corps d'une mère correspondant au corps humain.

11. Appareil d'inspection par ultrasons (1) selon l'une des revendications 1 à 10, dans lequel
l'unité d'affichage (26) est placée sur un côté du boîtier (11) de l'appareil d'inspection par ultrasons (1) opposé au côté sur lequel est placé l'unité de capteur par ultrasons (24).

12. Appareil d'inspection par ultrasons (1) selon l'une quelconque des revendications 1 à 11, comprenant en outre :
une unité de génération de données d'affichage (254) qui génère des données d'affichage pour rendre l'unité d'affichage (26) plus lumineuse, qui est fournie à une position correspondant à l'unité de réception ultrasonique (242) qui a reçu une onde ultrasonique réfléchie relativement forte, parmi la pluralité d'unités de réception ultrasonique (242).

13. Appareil d'inspection par ultrasons (1) selon la revendication 12, dans lequel
l'unité de génération de données d'affichage (254) génère les données d'affichage pour recommander le déplacement de l'appareil d'inspection par ultrasons (1) vers une position où se trouve l'unité de réception ultrasonique (242) qui a reçu une onde ultrasonique réfléchie d'intensité relativement élevée parmi la pluralité d'unités de réception ultrasonique (242).
